# EUROPEAN PATENT SPECIFICATION

(11) **EP 0 265 220 B1**
(45) Date of publication and mention of the grant of the patent: **07.01.1993**
(21) Application number: 87309240.7
(22) Date of filing: 20.10.1987
(51) Int. Cl.: C07C 69/16, C07C 67/42, C07C 45/54, C07C 49/345, C07C 67/08, C07C 45/46

(54) **Method for producing 2,6-diacetoxynaphthalene**
Herstellungsverfahren von 2,6-Diacetoxynaphthalen
Méthode de préparation du 2,6 diacétoxynaphtalène

(30) Priority: 20.10.1986 US 921702
(43) Date of publication of application: 27.04.1988
(73) Proprietor: HOECHST CELANESE CORPORATION, Somerville, N.J. 08876 (US)
(72) Inventor: Gerberich, Harold R., Corpus Christi Texas (US)
(74) Representative: De Minvielle-Devaux, Ian Benedict Peter

(56) References cited:
- EP-A- 0 178 929

## Description

The invention relates to an integrated method for producing 2,6-diacetoxynaphthalene (DAN).

With regard to the process disclosed and claimed herein, the oxidation of ketones with peroxy compounds to form esters, i.e. the "Baeyer-Villiger" reaction, is known in the art. Some references with disclosures or examples of this type of reaction and description of their teachings are as follows:
Hirao et al, Koen Yoshishu - Koryo, Terupen oyobi Seiyu Kagaku ni kansuru Toronkai, 23rd 1979, 131-3 (Japan). Chem. Soc. Japan: Tokyo, Japan, as abstracted in C.A. (1980), 93, 7777n, show the Fries rearrangement of phenyl acetate and the Freidel-Crafts acetylation of phenol to form para-hydroxyacetophenone which is then oxidized with hydrogen peroxide using sulfuric acid as catalyst to para-hydroxyphenyl acetate; the latter is then hydrolyzed with sulfuric acid in acetic acid to yield hydroquinone.

Von E. Doering et al, Journal of the American Chemical Society 72, 5515-5518 (1950) teach the Baeyer-Villiger oxidation of various aromatic ketones, including acetophenone and para-substituted benzophenones, to esters using peracetic acid as the oxidant and show that sulfuric acid has a "marked catalytic action" with regard to the yield and reaction time (page 5517, col. 1).

Ogata et al, Journal of Organic Chemistry 43, No. 12, 2417-2419 teach the Baeyer-Villiger oxidation of acetophenone and substituted acetophenones to the corresponding aryl acetates using permonophosphoric acid as oxidant and sulfuric acid as catalyst and show that the reaction rate is highly dependent on the acidity of the solution.

Starcher et al, Journal of the American Chemical Society, 80, 4079-4082 (1958) teach the synthesis of lactones by means of the Baeyer-Villiger oxidation of cyclic ketones using peracetic acid as oxidant. In the paragraph bridging pages 4079 and 4080, the authors state: "The absence of inorganic impurities, notably water, hydrogen peroxide, mineral acids and salts, reduced polymerization to a minimum during the reaction step and avoided many of the by-products which plagued previous investigators."

Adam et al, Journal of Organic Chemistry, 44, No. 26, 4969 (1979) describe the use of bis(trimethylsilyl) monoperoxysulfate as a Baeyer-Villiger oxidant in the oxidation of ketones to their corresponding esters. The authors state the shortcomings of a previously-used oxidant for this purpose, Caro's or monoperoxysulfuric acid, are "(i) the use of aqueous conditions, (ii) the presence of strong acid and (iii) undesirable side reactions."

In addition to Hirao et al discussed previously, other references showing the formation of hydroxyaromatic ketones by the Fries rearrangement of aromatic esters or the Friedel-Crafts acylation of phenols are also in the literature. Following are some of these references with descriptions of their teachings:

Davenport et al, U.S. Patent No. 4,524,217, issued June 18, 1985, disclose the preparation of hydroxy aromatic ketones by the Fries rearrangement of esters of phenolic compounds. The patent lists 2,6-naphthylene as a contemplated aromatic group.

Davenport, U.S. Patent No. 4,593,125, issued June 3, 1986, teaches the acylation of 2-substituted naphthalenes, e.g., 2-naphthol, with anhydrous hydrogen flouride as catalyst, to obtain 6-substituted-2-naphthones such as 6-hydroxy-2-acetonaphthone (6,2-HAN).

Lewis, U.S. Patent No. 2,833,825 shows the rearrangement of esters of phenolic compounds, e.g., beta-naphthol, to hydroxyaryl alkyl ketones using anhydrous hydrogen fluoride as catalyst. The working examples of this patent are limited to the rearrangement of esters of higher fatty acids.

Simons et al, Journal of the American Chemical Society, 62, 485 and 486 (1940) show the use of hydrogen fluoride as a condensing agent for various rearrangements and at page 486 show the Fries rearrangement of phenyl acetate to obtain p-hydroxyacetophenone.

Dann and Mylius in a dissertation included as part of a series of Reports from the Institute for Applied Chemistry of the University of Erlangen, received for publication on January 7, 1954 and published in Annalen der Chemie 587 Band, pages 1 to 15, show the rearrangement of phenyl acetate in hydrogen fluoride to 4-hydroxyacetophenone. They also report the same reaction carried out by K. Weichert as described in Angewandte Chemie 56, 338 (1943). In addition, Dann and Mylius disclose the formation of hydroxy aromatic ketones from rearrangements in hydrogen fluoride of m-cresyl acetate, p-cresyl acetate, and guaiacol acetate.

Dann and Mylius also disclose the reaction of phenol and glacial acetic acid in the presence of hydrogen fluoride to produce 4-hydroxyacetophenone. This reaction may be conventionally characterized as a Friedel-Crafts acetylation of phenol with acetic acid as the acetylating agent.

Simons et al, Journal of the American Chemical Society, 61, 1795 and 1796 (1939) teach the acylation of aromatic compounds using hydrogen fluoride as a condensing agent and in Table 1 on page 1796 show the acetylation of phenol with acetic acid to produce p-hydroxyacetophenone.

Muessdoerffer and Niederprum in German Offenlegungschrift 2,616,986, published October 27, 1977, disclose the acylation of phenols and substituted phenols with an acyl chloride in the presence of hydrogen fluoride to yield the 4-acyl derivative in high yield with high selectivity. The inventors disclose that 2-naphthol and 7-chloro-2-naphthol can be acylated according to their invention but do not teach any specific method for the acylation of the naphthol derivatives nor do they indicate what isomer or isomers are produced with such naphthol derivatives.

Copending application Serial No. 870,062, filed June 3, 1986 by Davenport discloses the preparation of 6-hydroxy-2-acetonaphthone (6,2-HAN) by the Fries rearrangement of 2-naphthyl acetate.

Parent application Serial No. 06/661,552, filed October 17, 1984 discloses a process for the production of aromatic diols and their carboxylate esters, including the step of subjecting a hydroxy or acyloxy aromatic ketone to a Baeyer-Villiger oxidation with peracetic acid containing less than 0.1 wt.% of sulfuric based on the initially-added peracetic acid to obtain a mono- or diester of an aromatic diol. The application lists 2,6-naphthylene as a contemplated aromatic group.

2,6-Diacetoxynaphthalene has utility as a monomer for the preparation of various high molecular weight polyesters and polyester-amides capable of forming an anisotropic melt phase and suitable for being formed into shaped articles such as moldings, fibers and films. Such polymers are disclosed, for example, in U.S. Patents Nos. 4,184,996; 4,339,375; and 4,355,134.

### SUMMARY OF THE INVENTION

In accordance with this invention, 2-6-diacetoxynaphthalene (DAN) is produced from 6-hydroxy-2-acetonaphthone (HAN) by a method including the steps in sequence of acetylating or esterifying the HAN hydroxyl group with an acetylating agent, e.g. acetic anhydride, to produce 6-acetoxy-2-acetonaphthone (AAN), and subjecting the AAN to a Baeyer-Villiger oxidation with a peroxy compound, such as peracetic acid, to produce the DAN. The reaction is carried out such that there is present no more than about 0.1 wt.% of a mineral acid such as sulfuric or phosphoric, based on the weight of initially added peroxy compound. The invention thus excludes the use in unmodified form of commercial peracetic acid solutions as the peroxy compound, which generally contain sulfuric acid in amounts greater than the maximum amounts defined by this invention.

The operability of the foregoing oxidation of AAN to DAN is unexpected since it has been found, for example, that HAN cannot be similarly oxidized to 6-acetoxy-2-naphthol.

The acetylation or esterification of HAN to produce AAN proceeds as in equation (I): 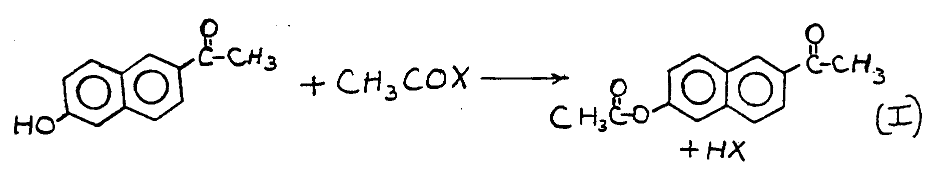
where X is the residue minus an acetyl group of compounds which are known acetylating agents. X may be, for example, hydroxy, acetoxy, or halide including fluoride, chloride, bromide, or iodide. Acetylating agents which may be used are for example, acetic anhydride, acetic acid, acetyl fluoride, acetyl chloride and acetyl bromide.

The Baeyer-Villiger oxidation of AAN with a peroxy compound to produce AAN proceeds as in equation (II):

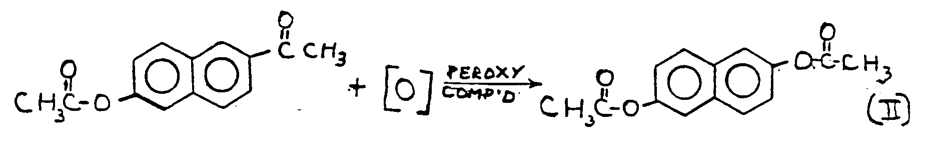

The HAN starting material for the production of DAN as hereindisclosed may be prepared by any method known in the art. Preferably, however, it is prepared by the Fries rearrangement of 2-naphthyl acetate as disclosed in previously cited U.S. application Serial No. 870,062, or by the Friedel-Crafts acetylation of 2-naphthol as taught by previously cited U.S. Patent No. 4,593,125. The entire disclosures of both the cited application and patent are incorporated herein by reference.

When producing DAN using 2-naphthyl acetate as the starting material, the initial Fries rearrangement to produce HAN from 2-naphthyl acetate is defined by equation (III):

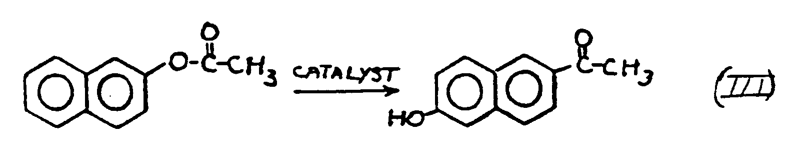

If 2-naphthol and an acetylating agent are used as the starting material, the resulting Friedel-Crafts acetylation reaction to form HAN is indicated by equation (IV):

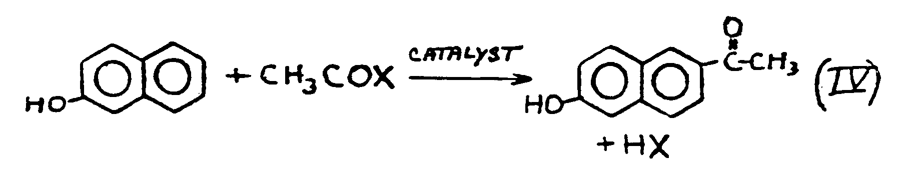

The Fries rearrangement or Friedel-Crafts catalyst for the foregoing reactions may be hydrogen fluoride or any other catalyst known in the art to be effective for the Fries or Friedel-Crafts reaction, e.g., aluminum chloride, zinc chloride, or boron trifluoride. In carrying out the reaction, the 2-naphthyl acetate, or 2-naphthol and acetylating agent, catalyst, and if desired when 2-naphthyl acetate is the starting material, an additive for the reaction for improvement of 6,2-HAN selectivity, such as acetic anhydride or acetic acid, may be charged to a corrosion-resistant reactor and the mixture maintained at a temperature, for example, of about 0 to about 100 degrees C, preferably about 50 to 80 degrees C for a period, for example, of 1/2 to about 8 hours, preferably about 1/2 to 4 hours, at a pressure, for example, of about 2.5 to about 500 psig. The acetic anhydride or acetic acid additive may be used, for example, in an amount of 0.1 to 2.0 moles, preferably 0.7 to 1.3 moles per mole of 2-naphthyl acetate.

If HF is used as the catalyst, it may be charged as a liquid or a gas using technologies of handling well known to those skilled in the art. In carrying out the reaction, an inert gas such as nitrogen may be used to keep the reaction space under the desired pressure and sufficient HF in contact with the reacting liquid. An excess of HF is generally used, for example, about 7 to about 75 moles, preferably, about 20 to 60 moles per mole of 2-naphthyl acetate or 2-naphthol initially present in the reaction zone. The reactants may be initially charged to the reactor. Hydrogen fluoride may then be charged at a temperature less than the specified reaction temperature and the reaction adjusted to the specified reaction temperature for the specified reaction period.

The 6-acetoxy-2-acetonaphthone (AAN) used as the starting material for the formation of DAN, may be obtained as a co-product with the HAN produced by the Fries rearrangement of 2-naphthyl acetate or the Friedel-Crafts acetylation of 2-naphthol, e.g., when the reaction is carried out in the presence of HF and acetic anhydride. Alternatively, it may be produced from the HAN by reacting the latter with an acetylating agent such as acetic anhydride, as shown in equation (I), by contacting the HAN with, for example, about 1 to 5 moles of the anhydride per mole of HAN at a temperature, for example, in the range of 120 to 140 degrees C for a period, for example, in the range of 1 to 4 hours.

In producing DAN in accordance with equation (II), the AAN is subjected to a Baeyer-Villiger oxidation by reacting it with a peroxy compound in the presence of no more than about 0.1 wt.% of a mineral acid based on the weight of initially added peroxy compound as shown in equation (II). The peroxy compound may be, for example, peracetic acid hydrogen peroxide, perbenzoic acid, m-chloroperbenzoic acid, trifluoroperacetic acid, permonophosphoric acid or bis(trimethylsilyl) monoperoxy-sulfate, or mixtures thereof, particularly, mixtures of a major amount of peracetic acid and a minor amount of hydrogen peroxide. The peroxy compound; e.g., peracetic acid, containing no more than about 0.1 wt.% of mineral acid may be obtained by removing by distillation or neutralization most of the mineral acid used as catylyst in the preparation of peroxy compounds and usually present in commercial mixtures, or may be prepared using as catalyst an acidic material which does not remain in the peroxy compound-containing product, e.g., an acidic ion-exchange resin.

The amount of peroxy compound, e.g. peracetic acid, supplied to the reaction may be in the range, for example, of about 0.5 to about 2 moles per mole of AAN. The reaction may be carried out at a temperature, for example, of about 20 to 140 degrees C and a pressure, for example, of about 25mmHgA to about 2 atmospheres for a period, for example, of about 0.5 to about 3 hours. A solvent for the AAN may be used in the initial reaction solution e.g. an alkanoic acid such as glacial acetic acid or formic acid.

Desirably, the Baeyer-Villiger oxidant is a purified peracetic acid which is contacted with the AAN under such conditions that the reaction solution contains no more than about 0.1 wt.%, preferably no more than about 0.05 wt.% of a mineral acid, e.g., sulfuric acid, based on the weight of pure peracetic acid initially added. In general, the less the mineral acid present the better. Preferably, the peracetic acid is added in the form of a solution consisting essentially of about 5 to 45 wt.% peracetic acid and the remainder a solvent such as acetic acid, formic acid, methyl acetate, ethyl acetate, acetone or mixtures thereof.

The following examples further illustrate the invention.

### DESCRIPTION OF SPECIFIC EMBODIMENTS

Unless otherwise identified, all pressures are Absolute.

### Example 1

This example illustrates the preparation of 6-hydroxy-2-acetonaphthone (HAN) by the Fries rearrangement of 2-naphthyl acetate using hydrogen fluoride as catalyst.

To a 300 cc Hastelloy C autoclave were charged 7.0 g (0.038 mol) of 2-naphthyl acetate. The autoclave was cooled to -50 degrees C and evacuated to 150mm Hg whereupon 33.3 moles of anhydrous hydrogen fluoride per mole of 2-naphthyl acetate were transferred from a cylinder to the autoclave at such a rate that the temperature did not exceed 0 degrees C. The contents were warmed to a reaction temperature of 75 degrees C and stirred for 4 hours during which time a pressure of ca. 40 psig was generated. At the end of the run, the hydrogen fluoride was vented through a caustic scrubber and the contents of the autoclave were poured onto ca. 30 g of ice. The pH of the mixture was adjusted to 6.5 using a solution of 50% potassium hydroxide and the mixture was then extracted with 75 ml of ethyl acetate (3x). The organic solution was dried over anhydrous MgSO₄, filtered, and the solvent was removed using a rotary evaporator to yield 6-hydroxy-2-acetonaphthone.

The conversion of 2-naphthyl acetate to all products was 99.7% while the selectivity to HAN based on all products was 67.6%, and the regioselectivity to HAN, which is the selectivity based on the total amount of hydroxacetonaphthone isomers produced was >99%.

### Example 2

This example illustrates the preparation of HAN by the Fries rearrangement of 2-naphthyl acetate using hydrogen fluoride as catalyst with acetic anhydride as additive.

The procedure of Example 1 was followed except that one mole of acetic anhydride per mole of 2-naphthyl acetate was charged to the reactor with the latter compound, 21.3 moles of hydrogen fluoride per mole of 2-naphthyl acetate were utilized and the reaction temperature was 60 degrees C.

The conversion of 2-naphthyl acetate was 95.2%, the selectivity to HAN based on all products was 54.8%, the selectivity to AAN was 20.1%, the regioselectivity to HAN was 98.6%, and the yield of HAN plus AAN was 71.3%.

### Example 3

This example illustrates the preparation of HAN by the Friedel-Crafts acetylation of 2-naphthol with acetic acid as the acetylating agent.

A solution of 14.4 g (0.1 mol) of 2-naphthol and 24.0 g (0. 4 mol) of acetic acid was cooled to -30 degrees C in a stainless steel autoclave. The solution was purged with 50 psig nitrogen for 15 minutes. Hydrogen fluoride, 100g (5.0 mol), was added and the autoclave sealed.

The autoclave was rapidly heated to 80 degrees C and maintained at that temperature for 60 minutes. The autoclave was then rapidly cooled to 40 degrees C. The hydrogen fluoride was purged from the autoclave at about 40 degrees C and then a nitrogen sweep was maintained for an additional one hour to remove the last distillable traces of hydrogen fluoride. The product was dissolved in ethyl acetate, poured onto ice, neutralized with the aqueous solution containing 45% potassium hydroxide until the aqueous solution was about pH 6.5. The aqueous layer was re-extracted with ethyl acetate. The organic layers were combined, washed with a saturated aqueous sodium chloride solution, dried, and the solvent was removed in vacuo.

The conversion of 2-naphthol to all products was 93% while the selectivity to HAN plus AAN was 76%.

### Example 4

This example illustrates the preparation of HAN by the Friedel-Crafts acetylation of 2-naphthol with acetic anhydride as the acetylating agent.

A solution of 14.4 g (0.1 mol) of 2-naphthol and 100 g (5.0 mol) of hydrogen fluoride is prepared at -30 degrees C. Acetic anhydride in an amount of 20.4 g (0.20 mole) was added and the reaction autoclave was sealed. The autoclave was heated rapidly to 80 degrees C and held at that temperature for one hour. Isolation and purification of the resulting HAN and its acetate were carried out as in Example 3. The conversion of 2-naphthol to all products was 99% and the selectivity to HAN plus AAN was greater than 85%.

### Example 5

This example illustrates the reaction of HAN with acetic anhydride to form AAN.

A solution of 186.2 g (1.0 mol) of HAN and 400 ml of acetic anhydride is heated at reflux for 3 h under a nitrogen atmosphere. The acetic acid and acetic anhydride are distilled overhead in vacuo (39-41 degrees C, 2.6 mm Hg). The remaining oil crystallizes upon cooling to yield 228.0 g (>99%) of white crystals identified as 6-acetoxy-2-acetonaphthone (AAN).

### Example 6

This and the following example illustrate the formation of DAN by the Baeyer-Villiger oxidation of AAN using as oxidant peracetic acid prepared by reacting hydrogen peroxide and acetic acid using an acidic ion exchange resin as catalyst.

To a 500 ml Erlenmeyer flask was added 180 grams of glacial acetic acid, 48.6 grams of 64 wt.% hydrogen peroxide (H₂O₂), and 3.0 grams of Amberlyst-15 sulfonic acid ion exchange resin made by Rohm and Haas. The mixture was agitated with a magnetic stirring bar at room temperature. After 6 hours, the peracetic acid concentration was 21.0 wt.%, which corresponded to 70% conversion of H₂O₂. After 24 hours, the peracetic acid concentration was 22.6 wt.% (76% H₂O₂ convervsion). The solution (24 hours) was filtered to separate the resin. The filtrate was used without further treatment and contained 2.5 wt.% of H₂O₂.

The oxidation of AAN was carried out using apparatus consisting of 250 ml three-neck round-bottomed flask to which was attached a 50 ml dropping funnel for the peracetic acid feed, a thermometer, and a condenser/vacuum pump. To the reaction flask were added 18.0 grams of crude AAN (containing 85.6 wt.% of pure AAN and 2.4 wt.% of HAN) and 46.0 grams of glacial acetic acid. 36.4 Grams of the aforementioned peracetic acid were placed in the dropping funnel. The AAN solution was heated to boiling (75 degrees C) under a vacuum of 215 mmHgA. The peracetic acid was then added drop-wise over 20 minutes. The solution was heated at 75 degrees C for an additional 340 minutes.

The product was cooled to room temperature to crystallize DAN. The precipitate was filtered and dried. Analysis of the dry solid and filtrate showed that the conversion of AAN was 57.2% and the yield of DAN was 46.6%. The DAN efficiency was 81.5%.

### Example 7

The procedure of Example 6 was followed except that the oxidation was carried out using 9.0 grams of crude AAN containing 88.7 wt.% of pure AAN, 80.0 grams of glacial acetic acid and 29.4 grams of the described peracetic acid. The conversion of AAN was 45.6%, the yield of DAN was 30.2% and the DAN efficiency was 66.2%.

Examples 8 to 11 illustrate the production of DAN by oxidation of AAN with anhydrous peracetic acid substantially free of mineral acid and formed by distilling commercial peracetic acid containing about 40 wt.% of pure peracetic acid, about 6 wt. % of hydrogen peroxide, about 13 wt.% of water and about 1 wt.% of sulfuric acid.

### Example 8

To 400 ml of the described commercial peracetic acid placed in a 2-liter flask were added 400 ml of acetic anhydride dropwise from an addition funnel. The reaction temperature was kept between 25-27 degrees C by running under reduced pressure. The reaction was quite exothermic and the reflux was required to remove the heat of reaction. After anhydride addition was complete, the vacuum was adjusted to 28 mm Hg and the peracetic/acetic acid mixture distilled. The pot temperature was 37 degrees C and the overhead 34-36 degrees C. A small forecut (20 ml) was discarded and then 500 ml of peracetic solution collected containing about 28 wt. % peracetic acid and the remainder acetic acid.

Pure AAN (0.75 mole) was dissolved in 450 ml of acetic acid and heated to 90 degrees C. To this were added 286 grams of the foregoing anhydrous solution of peracetic acid in acetic acid over a period of one hour and the mixture allowed to react for an additional hour. The reaction temperature of 90 degrees C was controlled by reducing the pressure until the boiling point of the solvent matched the desired temperature which also helped remove heat of reaction. Gas chromatographic analysis of the reaction mixture indicated a 68% yield of DAN. Approximately 60% of DAN crystallized out on cooling and, after two recrystallizations, 68 g of pure DAN were obtained, corresponding to a 37% yield. The remaining DAN and unconverted AAN could be recovered by distillation of the acetic acid.

### Example 9

Using the oxidation apparatus and procedure of Example 6, 15 grams of crude AAN containing 95.1 wt.% of AAN and 3.7 wt.% of HAN were mixed with 80 grams of glacial acetic acid to which were added 28.0 grams of an anhydrous solution of 21.7 wt.% of peracetic acid in acetic acid free of mineral acid and prepared by distilling commercial peracetic acid in a manner similar to that described in Example 8. The reaction was carried out by adding the peracetic acid solution dropwise over a period of 20 minutes to the AAN solution heated to 60 degrees C at a vacuum of 85 mm Hg, then heating the solution at 60 degrees C for an additional 100 minutes.

Gas chromatographic analysis of the reaction mixture indicated an AAN conversion of 7.8%, a DAN yield of 8.6% and a DAN efficiency of 100%.

### Example 10

The procedure of Example 9 was followed except that 35 grams of the anhydrous peracetic acid solution and 73 grams of glacial acetic acid were used. The AAN conversion was 7.0%, the DAN yield was 7.8% and the DAN efficiency was 100% as indicated by gas chromatographic analysis.

### Example 11

The procedure of Example 9 was followed except that the crude AAN contained 95.7 wt.% of pure AAN and 2.0 wt.% of HAN, and 50.6 grams of anhydrous peracetic acid solution and 50 grams of glacial acetic acid were used, the peracetic acid was added to the reactor in a staged addition, and the reaction temperature was 80 degrees C. In this procedure 27.6 grams of peracetic acid were initially added over a period of 20 minutes; after an interval of 40 minutes, 11.5 grams of solution were added over a period of 20 minutes; after another interval of 40 minutes, 11.5 grams of solution were added over a period of 20 minutes. The reaction was then continued at 80 degrees C and 200 mm Hg vacuum for a total reaction time of 180 minutes. Workup and analysis of the product as described in Example 6 indicated an AAN conversion of 98.0%, a DAN yield of 33.2% and a DAN efficiency of 33.9%.

Examples 12 to 19 illustrate the production of DAN by oxidation of AAN with a neutralized peracetic acid solution in acetic acid obtained by adding sodium acetate to a commercial peracetic acid solution having the same composition as that used to prepare the anhydrous peracetic acid solution employed in Examples 8 to 11. The sodium acetate was added in an amount sufficient to neutralize the sulfuric acid present. The neutralized solution contained 40 wt.% of peracetic acid, 6 wt.% of hydrogen peroxide, about 14 wt.% of water and a small amount of dissolved sodium sulfate with the remainder being acetic acid.

### Example 12

The oxidation procedure of Example 6 was followed except that the feed was 9 grams of crude AAN containing 95.7 wt.% AAN and 2.0 wt.% HAN, 80 grams of glacial acetic acid, and 14.5 grams of the described neutralized peracetic acid solution added dropwise over a 30 minute period, and the reaction was carried out at a temperature of 90 degrees C and under a vacuum of 395 mm Hg for a total time of 120 minutes. Workup and analysis of the product as described in Example 6 indicated an AAN conversion of 40.5%, a DAN yield of 24.1% and a DAN efficiency of 59.4%.

### Example 13

The procedure of Example 12 was followed except that 8.2 grams of crude AAN containing 79.3 wt.% of AAN and 0.85 wt.% of HAN were reacted, the neutralized peracetic acid solution was added dropwise over a 20 minute period, and the reaction was carried out at 75 degrees C under a vacuum of 210 mm Hg for a total time of 360 minutes. The AAN conversion was 72.8%, the DAN yield 69.7%, and the DAN efficiency 95.7%.

### Example 14

The procedure of Example 13 was followed except that 9.5 grams of crude AAN were reacted, and the oxidation was carried out by adding the neutralized peracetic acid solution to the reactor dropwise over a 20 minute period at a temperature of 60 degrees C and under 85 mm Hg vacuum, continuing the reaction at the latter conditions for an additional 40 minutes, continuing the reacting at 80 degrees C and 220 mm Hg vacuum for 120 minutes, and completing the reaction at 90 degrees C and 405 mm Hg vacuum for 60 minutes. The AAN conversion was 86.9%, the DAN yield 63.3% and the DAN efficiency 72.8%.

### Example 15

The procedure of Example 13 was followed except that 9.0 grams of crude AAN containing 85.9 wt.% of AAN and 1.3 wt.% of HAN were reacted and the oxidation was carried out by adding the neutralized peracetic acid solution to the reactor dropwise over a 20 minute period at 90 degrees C and 400 mm Hg vacuum, continuing the reaction at the latter conditions for 40 minutes, and completing the reaction at 75 degrees C for 240 minutes. The AAN conversion was 78.2%, the DAN yield 68.2%, and the DAN efficiency 87.2%.

### Example 16

The procedure of Example 15 was followed except that the neutralized peracetic acid solution was added to the reactor dropwise over a 1 minute period and the reaction was carried out at 25 degrees C and atmospheric pressure for a total reaction time of 169 hours. The AAN conversion was 63.5% and the DAN yield was 68.2%.

### Example 17

The procedure of Example 12 was followed except that 15 grams of crude AAN containing 85.6 wt.% of AAN and 2.4 wt.% of HAN, 66 grams of glacial acetic acid and 18.7 grams of neutralized peracetic acid added dropwise over a 20 minute period were used, and the reaction was carried out at 112.6 degrees C and atmospheric pressure for a total reaction time of 60 minutes. Gas chomatographic analysis of the crude product indicated an AAN conversion of 65.5%, a DAN yield of 48.8% and a DAN efficiency of 74.5%.

### Example 18

The procedure of Example 17 was followed except that 18 grams of crude AAN, 60 grams of glacial acetic acid, and 22.4 grams of neutralized peracetic acid solution were used, and the reaction was carried out at 80 degrees C and 210 mm Hg vacuum for a total reaction time of 630 minutes. Workup and analysis of the product as described in Example 6 indicated an AAN conversion of 76.8%, a DAN yield of 49.6% and a DAN efficiency of 64.5%

### Example 19

The procedure of Example 18 was followed except that the reaction was carried out at a temperature of 75 degrees C under 215 mm Hg vacuum for a total reaction period of 360 minutes. The AAN conversion was 79.4%, the DAN yield 57.2% and the DAN efficiency 77.8%.

### Example 20

This example illustrates the use of m-chloroperbenzoic acid as the oxidant in the Baeyer-Villiger oxidation.

The oxidation and analysis procedures of Example 6 were followed except that the feed was 8 grams of crude AAN, 80 grams of glacial acetic acid and 6.4 grams of 80% m-chloroperbenzoic acid added all at once at the start of the reaction, and the reaction was carried out at a temperature of 60 degrees C under 115 mm Hg vacuum for a total reaction period of 240 minutes. The AAN conversion was 43% and the DAN yield was 53%.

### Example 21

This example illustrates the use of hydrogen peroxide as oxidant and 90% formic acid as the solvent in the Baeyer-Villiger oxidation.

The oxidation procedure of Example 11 was followed except that the glacial acetic acid was replaced by 93.4 grams of 90% formic acid, the peracetic acid was replaced by 6.6 grams of 50% hydrogen peroxide added dropwise over a 30 minute period, and the reaction was carried out at 60 degrees C under 140 mm Hg vacuum for a total reaction period of 120 minutes. Gas chromatographic analysis of the product indicated an AAN conversion of 49.8%, a DAN yield of 6.8% and a DAN efficiency of 12.6%.

### Comparative Example A

This example illustrates the inoperability of the invention when more than 1% of a mineral acid based on the weight of initially added peroxygen compound is present in the reaction.

The procedure of Example 21 was followed except that 85 grams of glacial acetic acid were utilized as the solvent in place of the 90% formic acid, 1.2 grams of 85% phosphoric acid were added as catalyst and 8.9 grams of 50% hydrogen peroxide were added to the reactor as oxidant in a staged addition, and the reaction was carried out at 85 degrees C under 295 mm Hg vacuum for a total reaction time of 180 minutes. Gas chromatographic analysis indicated an AAN conversion of 38.9%. However no amount of DAN was detected, as a result of the presence during the reaction of more than 0.1 wt.% of phosphoric acid based on the weight of initially added hydrogen peroxide.

### Comparative Example B

This example illustrates the inoperability of the invention as applied to the oxidation of HAN.

The oxidation and analytic procedures of Example 13 were followed except that the crude AAN was replaced with 9 grams of pure HAN, 13.8 grams of the neutralized peracetic acid solution were added dropwise in a 20 minute period, and the reaction was carried out at 75 degrees C under 215 mm Hg vacuum for a total reaction period of 240 minutes. Although the analysis indicated a HAN conversion of 45.6%, there was no detectable amount of DAN or 6-acetoxy-2-naphthol.

## Claims

1. A method of producing 2,6-diacetoxynaphthalene comprising subjecting 6-acetoxy-2-acetonaphthone to a Baeyer-Villiger oxidation using a peroxy compound as oxidant such that the reaction mass contains no more than about 0.1wt.% of a mineral acid based on the weight of pure peroxy compound initially present.

2. The method of claim 1 wherein said peroxy compound is peracetic acid.

3. The method of claim 1 or 2 wherein said 6-acetoxy-2-acetonaphthone (AAN) is produced by contacting 2-naphthyl acetate with a Fries rearrangement catalyst to form 6-hydroxy-2-acetonaphthone (HAN), and reacting said HAN with an acetylating agent to form said AAN.

4. The method of claim 3 wherein said Fries rearrangement catalyst is hydrogen fluoride.

5. The method of claim 1 or 2 wherein said 6-acetoxy-2-acetonaphthone (AAN) is produced by contacting 2-naphthol and an acetylating agent with a Friedel-Crafts catalyst to form 6-hydroxy-2-acetonaphthone (HAN), and reacting said HAN with an acetylating agent to form said AAN.

6. The method of claim 5 wherein said Friedel-Crafts catalyst is hydrogen fluoride.

7. The method of claim 3,4,5 or 6 wherein said acetylating agent is acetic anhydride.

## Patentansprüche

1. Verfahren zur Herstellung von 2,6-Diacetoxynaphthalin, umfassend die Durchführung einer Baeyer-Villiger-Oxidation von 6-Acetoxy-2-acetonaphthon unter Verwendung einer Peroxy-Verbindung als Oxidationsmittel in der Weise, daß die Reaktionsmasse nicht mehr als etwa 0,1 Gew.-% einer Mineralsäure, bezogen auf das Gewicht der zu Beginn anwesenden reinen Peroxy-Verbindung, enthält.

2. Verfahren nach Anspruch 1, worin die Peroxy-Verbindung Peressigsäure ist.

3. Verfahren nach Anspruch 1 oder 2, worin das 6-Acetoxy-2-acetonaphthon (AAN) durch In-Berührung-Bringen von 2-Naphthylacetat mit einem Katalysator für eine Friessche Umlagerung zur Bildung von 6-Hydroxy-2-acetonaphthon (HAN) und Umsetzung dieses HAN mit einem Acetylierungsmittel hergestellt wird, um AAN zu bilden.

4. Verfahren nach Anspruch 3, worin der Katalysator für die Friessche Umlagerung Hydrogenfluorid ist.

5. Verfahren nach Anspruch 1 oder 2, worin das 6-Acetoxy-2-acetonaphthon (AAN) durch In-Berührung-Bringen von 2-Naphthol und einem Acetylierungsmittel mit einem Friedel-Crafts-Katalysator zur Bildung von 6-Hydroxy-2-acetonaphthon (HAN) und Umsetzung dieses HAN mit einem Acetylierungsmittel hergestellt wird, um AAN zu bilden.

6. Verfahren nach Anspruch 5, worin der Friedel-Crafts-Katalysator Hydrogenfluorid ist.

7. Verfahren nach Anspruch 3, 4, 5 oder 6, worin das Acetylierungsmittel Essigsäureanhydrid ist.

## Revendications

1. Méthode de préparation de 2,6-diacétoxynaphtalène consistant à soumettre de la 6-acétoxy-2-acétonaphtone à une oxydation de Baeyer-Villiger en utilisant un composé peroxy comme oxydant de manière que la masse réactionnelle ne contienne pas plus d'environ 0,1% en poids d'un acide minéral en se basant sur le poids du composé peroxy pur initialement présent.

2. Méthode de la revendication 1, où ledit composé peroxy est l'acide peracétique.

3. Méthode de la revendication 1 la revendication 2, où ladite 6-acétoxy-2-acétonaphtone (AAN) est produite par mise en contact de 2-naphtyl acétate avec un catalyseur de réarrangement de Fries pour former la 6-hydroxy-2-acétonaphtone (HAN) et réaction de ladite HAN avec un agent acétylant pour former ladite AAN.

4. Méthode de la revendication 3, où ledit catalyseur de réarrangement de Fries est du fluorure d'hydrogène.

5. Méthode de la revendication 1 ou 2, où ladite 6-acétoxy-2-acétonaphtone (AAN) est produite par mise en contact du 2-naphtol et d'un agent acétylant avec un catalyseur de Friedel-Crafts pour donner de la 6-hydroxy-2-acétonaphtone (HAN) et réaction de ladite HAN avec un agent acétylant pour former ladite AAN.

6. Méthode de la revendication 5, où ledit catalyseur de Friedel-crafts est du fluorure d'hydrogène.

7. Méthode de la revendication 3, 4, 5 ou 6, où ledit agent acétylant est l'anhydride acétique.
